# EUROPEAN PATENT APPLICATION

(11) **EP 3 326 519 A1**
(43) Date of publication of application: **30.05.2018**
(21) Application number: 17202518.1
(22) Date of filing: 20.11.2017
(51) Int. Cl.: A61B 5/00

(54) **PHOTOACOUSTIC APPARATUS, CONTROL METHOD, AND PROGRAM**

(30) Priority: 25.11.2016 JP 2016229314; 09.08.2017 JP 2017154458
(71) Applicant: CANON KABUSHIKI KAISHA, Ohta-ku Tokyo 146-8501 (JP)
(72) Inventor: ABE, Naoto, Ohta-ku, Tokyo 146-8501 (JP)
(74) Representative: Houle, Timothy James

(57) **Abstract**

A photoacoustic apparatus according to an aspect of the present disclosure includes a hand-held type probe including a light source and a reception unit configured to receive a photoacoustic wave generated due to light irradiation of a subject with light emitted from the light source, and a supply unit configured to supply power to the light source in such a manner that a heat generation amount generated in the light source per unit time does not exceed a predetermined value.

## Description

### BACKGROUND OF THE INVENTION

### Field of the Invention

The present invention relates to an apparatus that acquires information based on light emitted from a light source.

### Description of the Related Art

In recent years, a photoacoustic apparatus configured to image an inside of a subject with use of the photoacoustic effect has been researched and developed as an imaging technique utilizing light. The photoacoustic imaging apparatus is an apparatus that generates an image of the inside of the subject with use of an ultrasonic wave (a photoacoustic wave) generated due to the photoacoustic effect from an optical absorber absorbing energy of light with which the subject is irradiated.

For the photoacoustic apparatus, a device provided in the form of a hand-held type probe and capable of easily accessing an observation site has also been researched and developed similarly to an ultrasonic diagnosis apparatus. Japanese Patent Application Laid-Open No. 2016-47077 discusses a photoacoustic imaging apparatus that includes a probe equipped with a light source unit and a reception unit built therein.

### SUMMARY OF THE INVENTION

A part of the power supplied to the light source for the light emission is converted into heat, and so the light source generates heat. In a case where the light source is built in a probe (in a case where the light source is disposed inside a housing), a temperature of the probe may increase due to the heat generation of the light source. Trouble may occur due to this increase in the temperature of the probe.

Therefore, the present invention is directed to an apparatus including a probe equipped with a light source built therein and capable of preventing or cutting down the increase in the temperature of the probe due to the heat generation of the light source.

According to a first aspect of the present invention, there is provided a photoacoustic apparatus as specified in claims 1 to 8. According to a second aspect of the present invention, there is provided a control method as specified in clams 9 to 13. According to a third aspect of the present invention, there is provided a program as specified in clam 14.

Further features of the present invention will become apparent from the following description of exemplary embodiments with reference to the attached drawings.

### BRIEF DESCRIPTION OF THE DRAWINGS

Fig. 1 is a block diagram of a photoacoustic apparatus according to a first exemplary embodiment.
Fig. 2 is a schematic view of a hand-held type probe according to the first exemplary embodiment.
Fig. 3 is a block diagram illustrating a computer and a peripheral configuration thereof according to the first exemplary embodiment.
Figs. 4A and 4B are timing charts according to the first exemplary embodiment.
Figs. 5A and 5B are timing charts according to a third exemplary embodiment.
Figs. 6A, 6B, and 6C are timing charts according to a fourth exemplary embodiment.

### DESCRIPTION OF THE EMBODIMENTS

Exemplary embodiments of the present invention relate to an apparatus that acquires information regarding an irradiation target by light irradiation. More specifically, the exemplary embodiments of the present invention relate to an apparatus that acquires photoacoustic image data derived from a photoacoustic wave generated from the light irradiation. An acoustic wave generated from the photoacoustic effect according to the exemplary embodiments of the present invention is typically an ultrasonic wave, and includes waves called a sound wave, an acoustic wave, and a photoacoustic wave.

The photoacoustic image data according to the exemplary embodiments of the present invention is a concept including any kind of image data derived from the photoacoustic wave generated from the light irradiation. For example, the photoacoustic image data is image data indicating a spatial distribution of at least one piece of subject information such as a generated sound pressure (initial sound pressure) of the photoacoustic wave, a density of optical absorption energy, an optical absorption coefficient, and a concentration of a substance forming a subject (e.g., oxygen saturation). The subject information acquired based on the photoacoustic wave generated from the light irradiation using a plurality of wavelengths different from each other or one another is spectral information such as the concentration of the substance forming the subject. The spectral information may be the oxygen saturation, a value acquired by weighting the oxygen saturation based on an intensity such as an absorption coefficient, a total hemoglobin concentration, an oxyhemoglobin concentration, or a deoxyhemoglobin concentration. Further, the spectral information may be a glucose concentration, a collagen concentration, a melanin concentration, or a volume fraction of fat or water.

Generally, as a light quantity of irradiation light is increased, a larger photoacoustic wave can be generated, and a reception signal of the photoacoustic wave has an improved signal-to-noise ratio (S/N). As a result, photoacoustic image data having a high image quality when being displayed can be acquired.

One conceivable configuration of the hand-held type probe of the photoacoustic apparatus is that the light source is disposed in a casing of the probe. Even in such a configuration, it is desirable to set the light quantity of the irradiation light to a large amount to display the high-quality photoacoustic image. However, a part of the power supplied to the light source is converted into heat, by which the light source generates heat. Typically, high power should be supplied to the light source to set the light quantity of the irradiation light to the large amount, and the heat generation amount of the light source also increases in this case. Here, the light quantity of the irradiation light (hereinafter also referred to as an irradiation light quantity) is defined to be a total amount of light energy of one pulse (the unit thereof is J (Joule)). Accordingly, average power of the irradiation light (the unit thereof is W (Watt)) is acquired by multiplying the irradiation light quantity by the number of times that the light is emitted per second. For example, in a case where a laser diode is used as the light source and the light is emitted by an irradiation light quantity of 0.01 [J] at a time interval of 0.1 seconds (in a case where the light is emitted 10 times in 1 second), the average power of the irradiation light is 0.01 [J] × 10 (number of times/s) = 0.1 [W]. Then, assuming that light conversion efficiency with respect to the supplied power is 10 [%], the light source generates heat by an amount of 0.9 [W] per unit time in the case where the average power is 0.1 [W], assuming that the supplied power is 1 [W]. In this example, it is assumed that power unconverted into the light that is a part of the power supplied to the light source is entirely converted into the heat. The light of one pulse includes light having any kind of waveform, such as a triangular wave and a sine wave, besides light having a light intensity changing in the form of a square wave over time.

It is difficult to include a cooling mechanism such as forced wind cooling and water cooling in the hand-held type probe. Therefore, even if the light source provided inside the housing generates the heat by a small amount, the temperature inside the housing may increase. A failure may occur in a device inside the housing due to this increase in the temperature. Further, the increase in the temperature of the housing may also cause an increase in a temperature of a contact surface in contact with a user of the probe, a patient who is the subject, or the like.

Therefore, the present inventor(s) has(have) found that controlling the power to be supplied to the light source so as to keep the heat generation amount of the light source at an allowable heat generation amount in consideration of a heat dissipation capability of the hand-held type probe, can keep the temperature of the probe under control. More specifically, the present inventor(s) has(have) found that controlling the power to be supplied to the light source so as to emit the light by an irradiation light quantity and at a light emission timing capable of keeping the heat generation amount of the light source at the allowable heat generation amount in consideration of the heat dissipation capability of the hand-held type probe, can keep the temperature of the probe under control. Typically, the power to be supplied to the light source is controlled in such a manner that a heat generation amount proportional to a value acquired by multiplying the irradiation light quantity by a repetition frequency falls within an allowable range.

Further, in a case where the subject is skin of a human body or the like, the light irradiation should obey a maximum permissible exposure (MPE). The power to be supplied to the light source may be limited in such a manner that the irradiation light quantity does not exceed the MPE, in addition to being limited in consideration of the heat generation amount per unit time.

A target to which the present invention is applied is not limited to a photoacoustic apparatus that will be described in the following exemplary embodiments. The present invention can be applied to any apparatus that includes a hand-held type probe equipped with a built-in light source. For example, the present invention may be applied to a hand-held type probe equipped with a built-in light source and a built-in light reception element that receives reflected light or transmitted light of the light emitted from the light source. In other words, the present invention may be applied to an apparatus that includes a hand-held type probe equipped with a built-in light source and an information acquisition unit that acquires information regarding an irradiation target based on a reception signal of light propagating through the irradiation target.

In the following description, representative exemplary embodiments of the present invention will be described with reference to the drawings. However, dimensions, materials, and shapes of components that will be described below, a relative layout among them, and the like shall be changed as appropriate according to a configuration of the apparatus to which the present invention is applied and various kinds of conditions, and are not intended to limit the scope of the present invention to the following description. Each of the embodiments of the present invention described below can be implemented solely or as a combination of a plurality of the embodiments or features thereof where necessary or where the combination of elements or features from individual embodiments in a single embodiment is beneficial.

### <Configuration of Apparatus>

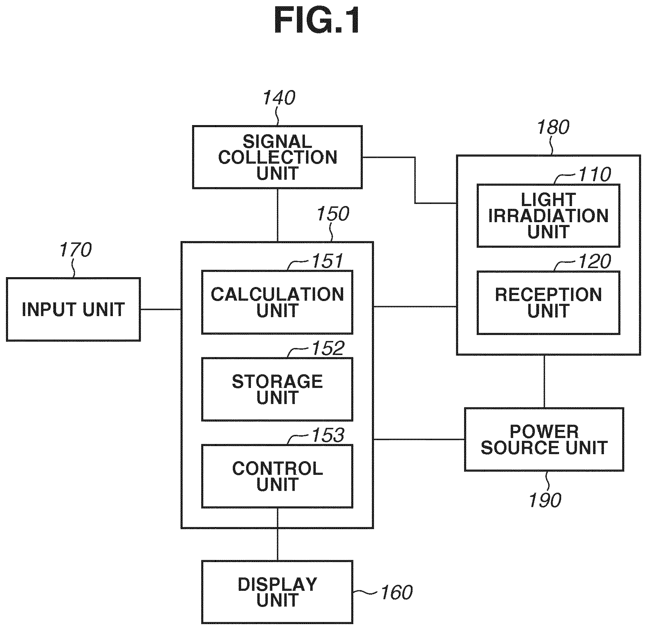

In the following description, a configuration of a photoacoustic apparatus according to a first exemplary embodiment will be described with reference to Fig. 1. Fig. 1 is a schematic block diagram of the entire photoacoustic apparatus. The photoacoustic apparatus according to the present exemplary embodiment includes a probe 180 (a light irradiation unit 110 and a reception unit 120), a signal collection unit 140, a computer 150, a display unit 160, an input unit 170, and a power source unit 190.

The light irradiation unit 110 irradiates a subject 100 with light, and an acoustic wave is generated from the subject 100. The acoustic wave generated from the photoacoustic effect due to the light will also be referred to as a photoacoustic wave. The power source unit 190 supplies power for driving a light source of the light irradiation unit 110. The reception unit 120 outputs an electric signal (photoacoustic signal) as an analog signal by receiving the photoacoustic wave.

The signal collection unit 140 converts the analog signal output from the reception unit 120 into a digital signal, and outputs the converted signal to the computer 150. The computer 150 stores the digital signal output from the signal collection unit 140 as signal data derived from the photoacoustic wave.

The computer 150 generates image data by performing processing that will be described below on the stored digital signal. Further, the computer 150 outputs the image data to the display unit 160 after performing image processing for presenting a display on the acquired image data. The display unit 160 displays a photoacoustic image. A medical doctor, a technician, or the like as a user can make a diagnosis by confirming the photoacoustic image displayed on the display unit 160. The displayed image is stored into a memory in the computer 150, a data management system connected to a modality via a network, and/or the like based on a storage instruction from the user or the computer 150.

As a reconstruction algorithm for converting the signal data into three-dimensional volume data, the photoacoustic apparatus can employ any kind of method, such as the back projection method in the time domain, the back projection method in the Fourier domain, and the model-based method (repetitive calculation method). Examples of the back projection method in the time domain include Universal Back-projection (UBP), Filtered Back-projection (FBP), and phasing and summing (Delay-and-Sum).

Further, the computer 150 also controls driving of the configuration included in the photoacoustic apparatus. Further, the display unit 160 may display a graphical user interface (GUI) and/or the like in addition to the image generated by the computer 150. The input unit 170 is configured to allow the user to input information. The user can perform an operation such as an instruction to start or end measurement, or store the generated image with use of the input unit 170.

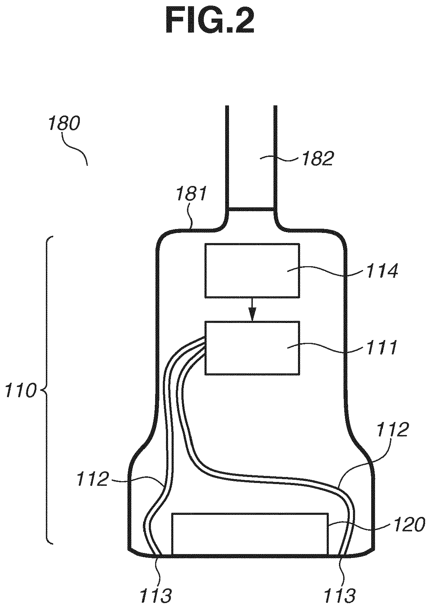

Fig. 2 is a schematic view of the hand-held type probe 180 according to the present exemplary embodiment. The probe 180 includes the light irradiation unit 110, the reception unit 120, and a housing 181. The housing 181 is a casing surrounding the light irradiation unit 110 and the reception unit 120. The user can use the probe 180 as the hand-held type probe by holding the housing 181. The light irradiation unit 110 includes a light source 111, an optical system 112, which causes the light generated from the light source 111 to propagate, and a driver circuit 114 for driving the light source 111. The light is emitted from an emission end 113 of the optical system 112 of the light irradiation unit 110. The optical system 112 illustrated in Fig. 2 transmits the light generated from the light source 111, such as a light-emitting diode (LED) and a laser diode (LD). The probe 180 is connected to the signal collection unit 140, the computer 150, and the power source unit 190 via a cable 182. The cable 182 includes a wiring for supplying power from the power source unit 190 to the driver circuit 114, a wiring for transmitting a control signal controlling an irradiation light quantity, a light emission timing, and the like from a control unit 153 to the driver circuit 114, and a wiring for outputting the analog signal that is the output from the reception unit 120 to the signal collection unit 140. The photoacoustic apparatus may be configured in such a manner that a connector is provided to the cable 182, and the probe 180 and the other configuration of the photoacoustic apparatus can be separated from each other. In the present exemplary embodiment, a configuration as a combination of the driver circuit 114 and the power source unit 190 corresponds to a supply unit that supplies power to the light source 111. In other words, the supply unit according to the present exemplary embodiment includes the driver circuit 114 and the power source unit 190.

The probe 180 according to the present exemplary embodiment may be a wireless hand-held type probe 180 with the cable 182 removed therefrom. In this case, the power source unit 190 may be incorporated in the probe 180, and the probe 180 may wirelessly transmit/receive various kinds of signals between the probe 180 and the other configuration. However, incorporating the power source unit 190 into the probe 180 leads to an increase in heat generation amount inside the housing 181 because of heat generated due to power consumption in the power source unit 190. Therefore, the power source unit 190 may be disposed outside the housing 181 to prevent or cut down an increase in a temperature inside the housing 181. Further, a partial configuration of the driver circuit 114 that consumes a large amount of power and generates a large amount of heat may be disposed outside the housing 181.

In the following description, each of the units in the photoacoustic apparatus according to the present exemplary embodiment will be described in detail.

### <Light Irradiation Unit 110>

The light irradiation unit 110 includes the light source 111, the optical system 112, and the driver circuit 114.

The light source 111 may be configured to include at least one of the LD and the LED. Further, the light source 111 may be a light source capable of changing a wavelength.

A pulse width of the light emitted by the light source 111 may be a pulse width of 1 ns or more and 1000 ns or less. Further, a wavelength of the light may be a wavelength that falls within a range approximately from 400 nm to 1600 nm. In a case where a blood vessel is imaged at a high resolution, the photoacoustic apparatus may employ a wavelength highly absorbable by the blood vessel (400 nm or longer and 700 nm or shorter). In a case where a deep portion in a living body is imaged, the photoacoustic apparatus may employ light having a wavelength typically little absorbable at a background tissue of the living body (water, fat, and the like) (700 nm or longer and 1100 nm or shorter).

An LD or an LED capable of emitting the light while following saw-tooth driving waveform (driving current) of 1 MHz or higher may be employed as the light source 111.

An optical element such as a lens, a mirror, and an optical fiber can be used for the optical system 112. In a case where a breast or the like is treated as the subject 100, a light emission portion of the optical system 112 may be formed by a diffusing plate or the like that diffuses the light to irradiate the subject 100 while expanding a beam diameter of the pulsed light. On the other hand, in a case where the photoacoustic apparatus functions as a photoacoustic microscope, the emission end 113 of the optical system 112 may be formed by a lens or the like to allow the subject 100 to be irradiated while the beam is focused thereon so as to increase a resolution. The light irradiation unit 110 may directly irradiate the subject 100 with the light from the light source 111 without including the optical system 112.

The driver circuit 114 is a circuit that generates the driving current for driving the light source 111 with use of the power from the power source unit 190.

### <Reception Unit 120>

The reception unit 120 includes a transducer that outputs the electric signal by receiving the acoustic wave, and a support member that supports the transducer.

A piezoelectric ceramic material represented by lead zirconate titanate (PZT), a polymer piezoelectric film material represented by polyvinylidene fluoride (PVDF), and the like can be used as a member for forming the transducer. Alternatively, an element other than the piezoelectric element may be also used. For example, a capacitive transducer (capacitive micro-machined ultrasonic transducer (CMUT)), a transducer using a Fabry-Perot interferometer, and the like can be used. Any transducer may be employed as long as this transducer can output the electric signal by receiving the acoustic wave. Further, the signal acquired by the transducer is a time-resolved signal. In other words, an amplitude of the signal acquired by the transducer indicates a value based on a sound pressure (e.g., a value proportional to the sound pressure) received by the transducer at each time.

A frequency component forming the photoacoustic wave is typically 100 KHz to 100 MHz, and an element capable of detecting these frequencies can be employed as the transducer.

The support member may be configured in such a manner that a plurality of transducers is disposed so as to be arrayed on a flat surface or a curved surface like an array called a 1 dimensional (D) array, a 1.5 D array, a 1.75 D array, or a 2 D array.

Further, the reception unit 120 may include an amplifier that amplifies the chronological analog signal output from the transducer. Further, the reception unit 120 may include an analog-to-digital (A/D) converter that converts the chronological analog signal output from the transducer into a chronological digital signal. In other words, the reception unit 120 may include the signal collection unit 140, which will be described below.

Ideally, the transducer may be arranged so as to surround the subject 100 from all around the subject 100 to allow the acoustic wave to be detected from various angles. However, in a case where the transducer cannot be arranged so as to broadly surround the subject 100 from all around the subject 100, the transducer may be arranged on a semispherical support member to make the layout thereof closer to a state where the subject 100 is surrounded from all around the subject 100. The layout and the number of the transducer(s), and the shape of the support member can be selected in any manner as long as they are optimized according to the subject 100, and any kind of reception unit 120 can be employed regarding the present exemplary embodiment.

A space between the reception unit 120 and the subject 100 may be filled with a medium that allows the photoacoustic wave to propagate therethrough. A material employable for this medium is a material that allows the acoustic wave to propagate therethrough, allows acoustic characteristics to match each other at an interface between the subject 100 and the transducer, and allows the photoacoustic wave to be transmitted therethrough at as a high transmittance as possible. For example, water or an ultrasonic gel can be employed as this medium.

Further, in a case where the apparatus according to the present exemplary embodiment also generates an ultrasonic image by the transmission and the reception of the acoustic wave in addition to the photoacoustic image, the transducer may function as a transmission unit that transmits the acoustic wave. The transducer as the reception unit and the transducer as the transmission unit may be a single (common) transducer or may be configured as different transducers.

### <Signal Collection Unit 140>

The signal collection unit 140 includes an amplifier that amplifies the electric signal that is the analog signal output from the reception unit 120, and an A/D converter that converts the analog signal output from the amplifier into the digital signal. The signal collection unit 140 may be constructed with use of a field programmable gate array (FPGA) chip or the like. The digital signal output from the signal collection unit 140 is stored into a storage unit 152 in the computer 500. The signal collection unit 140 is also called a data acquisition system (DAS). The electric signal in the present disclosure is a concept including both the analog signal and the digital signal. The signal collection unit 140 may be connected to a light detection sensor mounted at the light emission portion of the light irradiation unit 110, and may start the processing thereof synchronously by being triggered by the emission of the light from the light irradiation unit 110. Alternatively, the signal collection unit 140 may start this processing synchronously by being triggered by an instruction issued with use of a freeze button or the like.

The probe 180 may include the signal collection unit 140 including the amplifier, the A/D converter, and the like. In other words, the signal collection unit 140 may be disposed inside the housing 181. Such a configuration allows information between the hand-held type probe 180 and the computer 150 to propagate as a digital signal, and therefore can improve a noise resistance property. Further, compared to the transmission of the analog signal, the use of the high-speed digital signal allows the hand-held type probe 180 to reduce the number of wirings, thereby improving operability of the hand-held type probe 180.

### <Computer 150>

The computer 150 as an information processing unit includes a calculation unit 151, the storage unit 152, and the control unit 153. A function of each of the units will be described at the time of a description of a processing flow.

A unit in charge of a calculation function as the calculation unit 151 can be constructed with use of a processor, such as a central processing unit (CPU) and a graphics processing unit (GPU), or an arithmetic circuit, such as an FPGA chip. These units may be constructed not only with use of a single processor or arithmetic circuit, but also with use of a plurality of processors or arithmetic circuits. The calculation unit 151 may process a reception signal by receiving various kinds of parameters, such as a sound speed of the subject 100 and a configuration of a holding portion, from the input unit 170.

The storage unit 152 can be constructed with use of a non-transitory storage medium, such as a read only memory (ROM), a magnetic disk, and a flash memory. Alternatively, the storage unit 152 may be a volatile medium, such as a random access memory (RAM). A storage medium storing a program is a non-transitory storage medium. The storage unit 152 may be constructed not only with use of a single storage medium, but also with use of a plurality of storage media.

The storage unit 152 can store photoacoustic image data generated by the calculation unit 151. Further, the storage unit 152 can also store a display image based on the photoacoustic image data.

The control unit 153 is constructed with use of an arithmetic element, such as a CPU. The control unit 153 controls an operation of each of the units of the photoacoustic apparatus. The control unit 153 may control each of the units of the photoacoustic apparatus by receiving an instruction signal issued in response to various kinds of operations, such as a start of the measurement, from the input unit 170. Further, the control unit 153 reads out a program code stored in the storage unit 152, and controls activation of each of the units of the photoacoustic apparatus.

The computer 150 may be a workstation designed specifically therefor. Further, each of the units of the computer 150 may be constructed with use of a different hardware device. Alternatively, at least a part of the units of the computer 150 may be constructed with use of a single hardware device.

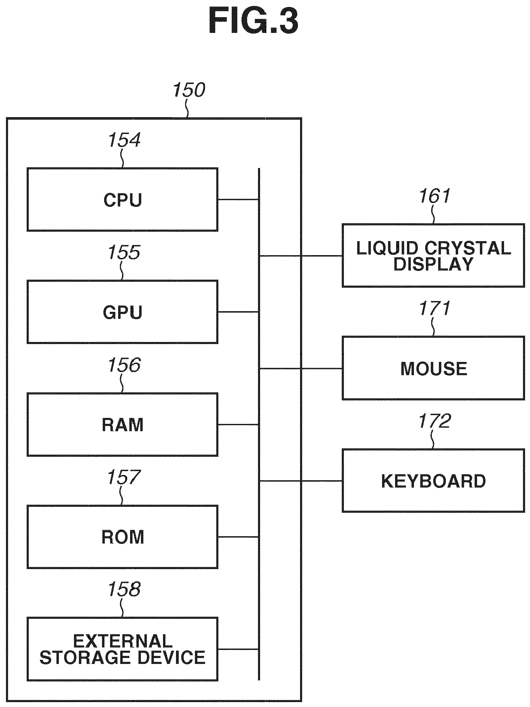

Fig. 3 illustrates an example of a specific configuration of the computer 150 according to the present exemplary embodiment. The computer 150 according to the present exemplary embodiment includes a CPU 154, a GPU 155, a RAM 156, a ROM 157, and an external storage device 158. Further, a liquid crystal display 161 as the display unit 160, a mouse 171 and a keyboard 172 as the input unit 170 are connected to the computer 150.

Further, the computer 150 and the reception unit 120 may be provided as a configuration being contained in a common casing. Further, the processing may be performed in such a manner that a computer contained in the casing performs a part of the signal processing and a computer disposed outside the casing performs the remaining signal processing. In this case, the computers disposed inside and outside the casing can be collectively regarded as the computer 150 according to the present exemplary embodiment. In other words, hardware forming the computer 150 does not have to be contained in a single casing.

### <Display Unit 160>

The display unit 160 is a display, such as a liquid crystal display and an organic electro luminescence (EL) display. The display unit 160 is a device that displays the image based on subject information and the like, a numerical value at a specific position, and the like acquired by the computer 150. The display unit 160 may display a GUI for operating the image and the apparatus. The display unit 160 can also present the display after the display unit 160 or the computer 150 performs image processing (e.g., adjustment of a luminance value) when displaying the subject information.

### <Input Unit 170>

An operation console including a mouse, a keyboard, and the like operable by the user can be employed as the input unit 170. Further, the display unit 160 may be constructed with use of a touch panel, thereby allowing the display unit 160 to be used as the input unit 170.

Each of the units of the photoacoustic apparatus may be configured as an individually different device, or may be configured as an integrated single device. Alternatively, at least a part of the units of the photoacoustic apparatus may be configured as an integrated single device.

### <Power Source Unit 190>

The power source unit 190 is a power source that generates power. The power source unit 190 supplies the power to the driver circuit 114 of the light irradiation unit 110. The power supplied from the power source unit 190 is consumed by, for example, the driver circuit 114 and the light source 111, and heat is generated along with the light emission. A direct-current (DC) power source can be used as the power source unit 190. Further, the power source unit 190 may be constructed with use of any kind of battery, such as a primary battery and a secondary battery. Constructing the power source unit 190 with use of the battery allows the power source unit 190 to be contained in the probe 180 in a space-saving manner. The driver circuit 114 and the power source unit 190 may be controlled by the control unit 153 in the computer 150. Further, the probe 180 may include a control unit that controls the power source unit 190 and the driver circuit 114.

### <Subject 100>

The subject 100 will be described below, although it is not a component forming the photoacoustic apparatus. The photoacoustic apparatus according to the present exemplary embodiment can be used for the purpose of a diagnosis, follow-up monitoring of a chemical treatment over time, or the like of a malignant tumor, a vascular disease, or the like of a human or an animal. Therefore, a site targeted for the diagnosis, such as a living body, in particular, a breast, each organ, a vascular network, a head, a neck, an abdomen, or four limbs including fingers and toes of the human or the animal, is assumed to be treated as the subject 100. For example, in a case where the measurement target is a human body, oxyhemoglobin or deoxyhemoglobin, a blood vessel including a large amount of them, a new blood vessel formed close to a tumor, or the like may be handled as an optical absorber target. Alternatively, plaque of a carotid artery wall or the like may be handled as the optical absorber target. Alternatively, a dye such as methylene blue (MB) and indocyanine green (ICG), gold fine particles, or an externally introduced substance acquired by collecting or chemically modifying them may be handed as the optical absorber. Alternatively, a puncture needle or an optical absorber stung by a puncture needle may be handled as an observation target.

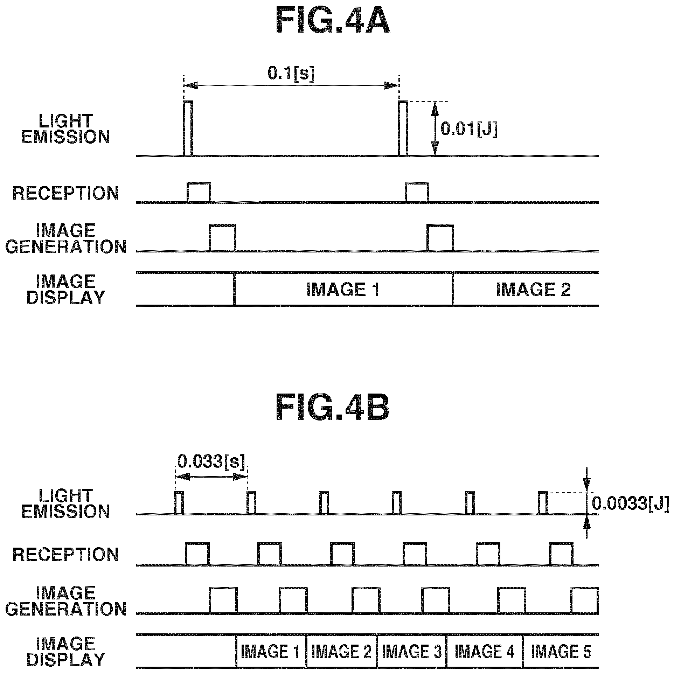

Figs. 4A and 4B are timing charts illustrating a method for controlling the irradiation light quantity of the light source 111 according to the present exemplary embodiment. Figs. 4A and 4B illustrate each of timings at which: the irradiation light is emitted, the photoacoustic wave is received, the image data is generated, and the image data is displayed. Axes of ordinate in the timing charts of the light emission indicate the irradiation light quantity. In Figs. 4A and 4B, the irradiation light quantity is assumed to be a value proportional to the power supplied to the light source 111.

First, the user specifies a display mode with use of the input unit 170. For example, Fig. 4A is a timing chart when a "real-time mode" is specified. A frequency at which the image display is refreshed in the "real-time mode" is 10 [Hz], which allows the display to keep up with a normal movement of the hand-held type probe 180. In the "real-time mode" illustrated in Fig. 4A, the frequency at which the image display is refreshed, and a frequency at which the light irradiation is repeated match each other.

The control unit 153 sets a light quantity setting value of 0.01 [J] to the driver circuit 114 and also outputs a signal indicating the light emission timing to the driver circuit 114 at a time interval of 0.1 seconds according to the specified display mode at a timing indicated by "light emission" illustrated in Fig. 4A. The driver circuit 114 drives the light source 111 according to the signal indicating the light emission timing and information indicating the setting value of the light quantity provided from the control unit 153.

Then, the reception unit 120 receives the photoacoustic wave generated due to the light output from the light source 111 at a timing indicated by "reception" illustrated in Fig. 4A. The calculation unit 151 performs reconstruction processing based on the signal output from the reception unit 120 to generate the image data at a timing indicated by "image generation" illustrated in Fig. 4A. Then, the control unit 153 transmits the image data to the display unit 160 to cause the display unit 160 to display the image thereon based on the image data. The display unit 160 displays the image based on the image data thereon for a time period indicated by "image display" illustrated in Fig. 4A.

In the "real-time mode" illustrated in Fig. 4A, first, an image 1 is displayed for 0.1 seconds, and, next, an image 2 is displayed for 0.1 seconds. This process is repeated, and an image display based on new image data is updated every 0.1 seconds.

As described above, for the hand-held type probe 180, an allowable irradiation light quantity of the light and an allowable number of times that the light is emitted (the frequency at which the light irradiation is repeated) per second are determined from a limit imposed on the heat generation amount per unit time. In the present exemplary embodiment, the control unit 153 controls the power to be supplied to the light source 111 in such a manner that the heat generation amount of the light source 111 per unit time falls below 1 [W]. In other words, the control unit 153 controls the power to be supplied to the light source 111 in such a manner that the heat generation amount of the light source 111 per unit time does not exceed a predetermined value (1 [W]). Assuming that the light conversion efficiency with respect to the supplied power is 10 [%], the average power of the irradiation light should fall below 0.11 [W] to keep the heat generation amount of the light source 111 per unit time under 1 [W]. Hereinafter, a calculation will be made, assuming that the light conversion efficiency with respect to the supplied power is 10 [%].

In the "real-time mode" according to the present exemplary embodiment, the average power is arranged to be limited to 0.1 [W] by setting the repetition frequency and the light quantity of one pulse to 10 [Hz] and 0.01 [J], respectively. This limitation can achieve a heat generation amount of 0.9 [W], thereby keeping the heat generation amount due to the light emission under the predetermined value (1W). The average power may exceed the predetermined value (0.11 [W]), if ignoring the heat generation amount and taking into consideration keeping the exposure amount under the maximum allowable exposure amount. However, it is assumed that the light irradiation may have to be carried out by a considerably smaller amount than the MPE, if taking into consideration the trouble due to the heat generation of the light source 111. The allowable heat generation amount per unit time may be set to a smaller value than 5 [W] to keep the temperature of the probe 180 at a temperature low enough for the user to hold the probe 180. Further, the allowable heat generation amount per unit time may be set to a smaller value than 3 [W] to prevent or cut down the increase in the temperature of the probe 180. Further, the allowable heat generation amount per unit time may be set to a smaller value than 1 [W] to prevent or cut down the increase in the temperature of the probe 180.

Next, a timing chart when the user specifies the "high-speed real-time mode" with use of the input unit 170 will be described with reference to Fig. 4B. The "high-speed real-time mode" is a display mode at a refresh frequency of 30 [Hz] higher than the refresh frequency of 10 [Hz] in the "real-time mode". According to this display mode, a further faster movement can be observed. In the "high-speed real-time mode" illustrated in Fig. 4B, the frequency at which the image display is refreshed and the frequency at which the light irradiation is repeated match each other.

If the light is emitted by the light quantity of 0.01 [J] similarly to the "real-time mode" in the case where the light is emitted at a repetition frequency of 30 [Hz] in conformity to the refresh frequency, heat is generated by a heat generation amount of 2.7 [W] per unit time, which undesirably exceeds the predetermined allowable value (1 [W]). Therefore, the control unit 153 controls the power to be supplied to the light source 111 in the "high-speed real-time mode" in such a manner that the light is emitted by a light quantity of 0.0033 [J], which is one-third of the irradiation light quantity in the "real-time mode", as indicated in "light emission" illustrated in Fig. 4B. Controlling the supplied power in this manner allows the heat generation amount per unit time to be reduced to 0.9 [W] similar to the "real-time mode".

The reception unit 120 receives the photoacoustic wave generated due to the light emitted from the light source 111 at a timing indicated by "reception" illustrated in Fig. 4B. The calculation unit 151 performs the reconstruction processing based on the signal output from the reception unit 120 to generate the image data at a timing indicated by "image generation" illustrated in Fig. 4B. Then, the control unit 153 transmits the image data to the display unit 160 to cause the display unit 160 to display the image based on the image data. The display unit 160 displays the image based on the image data thereon for a time period indicated by "image display" illustrated in Fig. 4B.

In the "high-speed real-time mode" illustrated in Fig. 4B, first, the image 1 is displayed for 0.033 seconds, and, next, the image 2 is displayed for 0.033 seconds. Similarly, an image 3, an image 4, and an image 5 are also displayed for 0.033 seconds for each of them sequentially. This process is repeated, and the image display based on the new image data is updated every 0.033 seconds.

In the "high-speed real-time mode", the refresh frequency increases, which necessitates a reduction in a time period required for the reconstruction processing. One possible method therefor is to use hardware simply improving a processing capability of the computer 150. Alternatively, the time period required for the reconstruction processing may be reduced by reducing a calculation amount for the reconstruction in the "high-speed real-time mode". For example, the calculation amount may be reduced by reducing a data amount by employing a coarse pitch for a reconstruction voxel, reducing the number of tones to be processed by the analog-digital conversion, or reducing the frequency to be processed by the analog-digital conversion.

Further, the irradiation light quantity decreases in the "high-speed real-time mode", which makes it difficult to deliver the light to a deep layer portion inside the subject 100. This means a reduction in usefulness of a photoacoustic wave reaching the reception unit 120 at a late timing from the light irradiation among photoacoustic waves reaching the reception unit 120. Therefore, the control unit 153 may set a short time period as a time period for the analog-digital conversion (a time period during which the photoacoustic wave is received) to reduce the number of data pieces, i.e., the data amount to be processed by the analog-digital conversion, thereby reducing the calculation amount. In this case, a narrower region (a region that the light sufficiently reaches) than compared to the "real-time mode" may be reconstructed. In this case, the photoacoustic apparatus can reduce both the time period during which the photoacoustic wave is received (corresponding to the "reception" illustrated in Figs. 4A and 4B), and the time period required for the image generation (corresponding to the "image generation" illustrated in Figs. 4A and 4B).

Between the "real-time mode" and the "high-speed real-time mode", the irradiation light quantity is different, and therefore the generated sound pressure of the photoacoustic wave changes and accordingly a magnitude of the reception signal changes. As a result, brightness of the displayed image is supposed to change. Displaying the reconstructed image in this manner impedes the user's observation of the reconstructed image.

Since the setting value of the light quantity is determined according to the mode, a ratio of the sound pressures of the photoacoustic waves that is generated between the modes is known in advance. Therefore, the computer 150 may make a correction so as to prevent the brightness of the displayed image of the image data from changing based on the setting value of the light quantity even when the light quantity of the light emitted from the light source 111 changes. A target for the correction may be any of the reception signal, the image data, and the display image. For example, supposing that the irradiation light quantity reduces to 1/3 when the mode is switched from the "real-time mode" to the "high-speed real-time mode", the correction in this case will be described now. In this case, the reception signal can be expected to reduce to 1/3. Therefore, the control unit 153 may triple a degree of amplification at which the analog output (photoacoustic signal) of the reception unit 120 is amplified, reduce a voltage range to be converted by the A/D converter to 1/3, or multiply the digital signal converted by the A/D converter by 3. The control unit 153 may perform at least one kind of processing among these kinds of processing, thereby performing processing that prevents the brightness of the displayed image from changing regardless of the set light quantity value.

Emitting the light by the irradiation light quantity set in the "high-speed real-time mode" even in the "real-time mode" allows the heat generation amount per unit time to fall below the predetermined value, and also eliminates a trouble of changing the setting of the irradiation light quantity between the modes. However, designing the photoacoustic apparatus in this manner leads to a reduction in the light quantity per unit time with respect to the light from the light irradiation unit 110 in the "real-time mode", and thus a reduction in the image quality of the displayed image in the "real-time mode". Therefore, it is desirable to control the supplied power in such a manner that the heat generation amount per unit time falls below the predetermined value and a difference in the light quantity per unit time also reduces between the modes.

In the display modes illustrated in Figs. 4A and 4B, the computer 150 generates and displays the image data every time the subject is irradiated with the light of one pulse. The computer 150 may generate and display the image data with use of reception signals of photoacoustic waves due to the light irradiation using a plurality of pulses. In other words, the frequency at which the light source 111 repeats the light emission and the frequency at which the image display is refreshed do not have to match each other. The frequency at which the image display is refreshed may be lower than the frequency at which the light source 111 repeats the light emission. Further, when an image of one frame is generated, a reception signal used when an image of another frame is generated may be used. In this case, a plurality of reception signals may be averaged, or a moving average thereof may be acquired.

As described above, according to the present exemplary embodiment, the user can select the "real-time mode" (first mode) in which the light irradiation is repeated at the frequency of 10 [Hz] (first repetition frequency). Further, the user can select the "high-speed real-time mode" (second mode) in which the light irradiation is repeated at the frequency of 30 [Hz] (second repetition frequency) higher than 10 Hz. Further, the user can switch the "real-time mode" and "high-speed real-time mode". Further, the power to be supplied to the light source 111 is controlled in such a manner that the light is emitted from the light source 111 by a first light quantity (0.01 [J]) when the subject 100 is irradiated with the light at the first repetition frequency (10 [Hz]). Further, the power to be supplied to the light source 111 is controlled in such a manner that the light is emitted from the light source 111 by a second light quantity (0.0033 [J]) smaller than the first light quantity when the subject 100 is irradiated with the light at the second repetition frequency (30 [Hz]).

The light irradiation unit 110 may include a plurality of light sources, and switch the light source 111 according to the mode. For example, the LD may be used as the light source 111 in the "real-time mode" in which the light quantity is large. On the other hand, the LED may be used as the light source 111 in the "high-speed real-time mode" in which the light quantity is small. By switching and using the light source capable of efficiently emitting the light quantity of each of the modes in this manner, the photoacoustic apparatus can efficiently use the supplied power, thereby leading to a reduction in the heat generation. Further, the photoacoustic apparatus can prevent the heat from being locally concentrated by switching the light source 111 according to the mode in this manner.

Further, the light irradiation unit 110 may expand an area to be irradiated with the light in the "high-speed real-time mode" while narrowing this area in the "real-time mode". In other words, the light irradiation unit 110 may increase a density of the light energy with which the subject 100 is irradiated by narrowing the irradiation area in the mode requiring a large light quantity. As a result, the photoacoustic apparatus can acquire a high generated sound pressure of the photoacoustic wave in the "real-time mode", thereby further improving the image quality of the displayed image. Further, in a case where the image quality is supposed to be kept consistent between the modes, the photoacoustic apparatus may reduce the light quantity to reduce the heat generation in the "real-time mode".

Further, the photoacoustic apparatus may switch the mode to a display mode of transmitting the ultrasonic wave and receiving the reflected ultrasonic wave to image the subject 100 with use of the probe 180 according to the present exemplary embodiment ("ultrasonic (US) mode"). In this case, the photoacoustic apparatus may use the reception unit 120 of the probe 180 as the unit that transmits the ultrasonic wave, or may include another transducer as the unit that transmits the ultrasonic wave. Further, the probe 180 may include a transducer different from the reception unit 120, which is the reception unit that receives the photoacoustic wave, and may transmit and receive the ultrasonic wave with use of this transducer. In exemplary embodiments that will be described below, the photoacoustic apparatus can also switch the mode to the US mode in a similar manner.

Further, in addition to the control of the supplied power so as to reduce the heat generation like the present exemplary embodiment, the probe 180 may include a temperature sensor, and the control unit 153 may cause a notification unit to issue a notification indicating temperature information of the probe 180 based on an output of the temperature sensor. For example, the control unit 153 may cause the notification unit to issue a notification indicating a warning if determining that the temperature of the probe 180 (e.g., the temperature inside the housing 181) reaches or exceeds 43 degrees Celsius based on the output from the temperature sensor. Further, the control unit 153 may also issue a warning if determining that the temperature of the probe 180 reaches a temperature lower than 43 degrees Celsius (e.g., 41 degrees Celsius). The control unit 153 may issue the notification in a stepwise manner with respect to the temperature of the probe 180 estimated from the output of the temperature sensor in this manner. For example, a unit that issues the notification with use of a display lamp, a sound/voice, and/or the like may be employed as the notification unit, besides a unit that displays the temperature information of the probe 180 on the display unit 160. Further, the control unit 153 may control the power source unit 190 and driver circuit 114 so as to stop the supply of the power to the light source 111 if determining that the temperature of the probe 180 is higher than a predetermined value based on the output of the temperature sensor. In the exemplary embodiments that will be described below, the photoacoustic apparatus may also issue the notification or perform the control with use of the temperature sensor in a similar manner.

Next, a second exemplary embodiment of the present invention will be described. The first exemplary embodiment of the present invention is an exemplary embodiment in which the photoacoustic apparatus sets the frequency at which the light irradiation is repeated according to the specified display mode, and sets the light quantity of the irradiation light capable of reducing the heat generation of the probe 180 at the set frequency at which the light irradiation is repeated. The second exemplary embodiment is an exemplary embodiment in which the photoacoustic apparatus allows the user to directly specify the refresh frequency, and controls the light irradiation to the repetition frequency and the irradiation light quantity according to the specified refresh frequency. The present exemplary embodiment will be described referring to an example in which the repetition frequency matches the refresh frequency, but the refresh frequency may be lower than the repetition frequency.

An apparatus configuration according to the present exemplary embodiment is similar to that of the first exemplary embodiment illustrated in Figs. 1 and 2, and therefore a description thereof will be omitted below.

The user first specifies the refresh frequency with use of the input unit 170. For example, the user specifies 10 [Hz] as the refresh frequency with use of the input unit 170. In this case, the user can observe the image updated at the refresh frequency of 10 [Hz] at the timings illustrated in Fig. 4A as if this image is a moving image. Even if the user does not specify the refresh frequency when instructing the photoacoustic apparatus to start the photoacoustic measurement, a default refresh frequency may be set.

Further, if the user desires to observe a faster movement, the user may specify a higher refresh frequency (e.g., 30 [Hz]) with use of the input unit 170. In this case, the user can observe the image updated at the refresh frequency of 30 [Hz] at the timings illustrated in Fig. 4B as if this image is a moving image.

Further, the user may specify a lower refresh frequency (e.g., 5 [Hz]). In this case, the control unit 153 also controls the power to be supplied to the light source 111 in such a manner that the heat generation amount per unit time falls below the predetermined value (1 [W]). When the repetition frequency is 5 [Hz], the light quantity of one pulse should fall below 0.022 [J]. Therefore, in the present exemplary embodiment, when the refresh frequency is set to 5 [Hz], the control unit 153 controls the power to be supplied to the light source 111 in such a manner that the repetition frequency matches 5 [Hz] and the light quantity of one pulse matches 0.02 [J]. By this control, the photoacoustic apparatus can keep the heat generation amount per unit time under the predetermined value, and further emit the light pulse by a larger irradiation light quantity than that when 10 [Hz] is specified as the refresh frequency. As a result, the photoacoustic apparatus can achieve a higher image quality of the displayed image than that when the refresh frequency is high.

In the second exemplary embodiment, if an extremely small value is specified as the refresh frequency, an extremely large value may be unintentionally set as the irradiation light quantity. However, the light irradiation is subjected to the limit value on the light quantity according to the MPE and an uppermost light quantity that the light source 111 such as the laser diode and the LED can emit (uppermost light quantity according to the device specifications). Therefore, the control unit 153 may control the power to be supplied to the light source 111 in such a manner that the light quantity emitted from the light source 111 falls below these upper limits.

The second exemplary embodiment has been described above based on the exemplary embodiment that allows the user to directly specify the refresh frequency with use of the input unit 170. The second exemplary embodiment will be described next based on an exemplary embodiment that automatically specifies the refresh frequency, as another exemplary embodiment of the second exemplary embodiment. This exemplary embodiment will also be described assuming that the frequency at which the light emission is repeated is determined according to the refresh frequency.

In the present exemplary embodiment, the hand-held type probe 180 includes a sensor that detects an orientation of the probe 180, such as an acceleration sensor and a gyroscope sensor. The computer 150 can estimate a moving speed of the hand-held type probe 180 based on an output from any of these sensors. Then, the control unit 153 determines the refresh frequency as will be described below, based on the estimated moving speed. Then, the control unit 153 controls the frequency at which the light emission is repeated and the irradiation light quantity according to the refresh frequency as described above.

Now, a position of an object appearing in the displayed image changes due to the movement of the hand-held type probe 180. Further, when the hand-held type probe 180 moves at a high (fast) moving speed, the position of the object appearing in the displayed image also considerably changes. Then, if the refresh frequency is low when the hand-held type probe 180 moves at the high speed, the object appearing in the displayed image may be unintentionally replaced with a different object when the displayed image is updated, and the diagnosis may be impeded thereby. Therefore, the control unit 153 sets the refresh frequency to a high frequency when the hand-held type probe 180 moves at the high speed based on a result of the detection of the orientation of the probe 180. On the other hand, the control unit 153 sets the refresh frequency to a low frequency when the hand-held type probe 180 moves at a low speed. Then, the control unit 153 determines the frequency at which the light emission is repeated and the irradiation light quantity as described above according to the refresh frequency set based on the speed of the probe 180. An upper limit may be imposed on the refresh frequency in consideration of a human cognitive capacity and a signal processing capability of the computer 150 even when the hand-held type probe 180 moves at an extremely high speed. For example, the upper limit on the refresh frequency may be set to 240 [Hz]. In this manner, when the hand-held type probe 180 moves at the high speed, the irradiation light quantity decreases while the refresh frequency increases and the repetition frequency increases. As a result, the image quality of the reconstructed image decreases. However, the human visual perception is characterized by having a difficulty in determining a detailed portion with respect to a swiftly moving object but being able to excellently determine a detailed portion when observing a slowly moving object or a stationary object. Therefore, the user little feels interrupted from the reduction in the image quality of the reconstructed image when the hand-held type probe 180 moves at the high speed. On the other hand, when wanting to view the details of the reconstructed image, the user observes the object while slowing down the speed of the hand-held type probe 180, which causes the control unit 153 to increase the setting value of the light irradiation amount, so that a high-quality display image can be provided.

The light irradiation unit 110 may include a plurality of light sources, and switch the light source 111 according to the frequency at which the light emission is repeated or the irradiation light quantity, which are determined according to the refresh frequency. For example, the LD may be used as the light source 111 when the refresh frequency is lower than a predetermined value and the irradiation light quantity is large. On the other hand, the LED may be used as the light source 111 when the refresh frequency is higher than the predetermined value and the irradiation light quantity is small. By switching and using the light source capable of efficiently emitting the irradiation light quantity according to the refresh frequency in this manner, the photoacoustic apparatus can efficiently use the supplied power, thereby leading to a reduction in the heat generation. Further, the photoacoustic apparatus can prevent the heat from being locally concentrated by switching the light source 111 according to the refresh frequency in this manner.

Further, the light irradiation unit 110 may expand the area to be irradiated with the light when the refresh frequency is high while narrowing this area when the refresh frequency is low. In other words, the light irradiation unit 110 may increase the density of the light energy with which the subject 100 is irradiated by narrowing the irradiation area at the time of the refresh frequency requiring a large irradiation light quantity. As a result, the photoacoustic apparatus can acquire a high generated sound pressure of the photoacoustic wave when the refresh frequency is low, thereby further improving the image quality of the displayed image. Further, in a case where the image quality is supposed to be kept consistent regardless of the refresh frequency, the photoacoustic apparatus may reduce the irradiation light quantity to reduce the heat generation when the refresh frequency is low.

Next, a third exemplary embodiment of the present invention will be described. The present exemplary embodiment will be described as a photoacoustic apparatus capable of switching the "real-time mode" of repeating the light irradiation to update the displayed image and a "freeze mode" of irradiating the subject 100 with the light to generate and display the image data at a timing specified by the user.

An apparatus configuration according to the present exemplary embodiment is similar to that according to the first exemplary embodiment illustrated in Figs. 1 and 2, and therefore a description thereof will be omitted below.

The "freeze mode" according to the present exemplary embodiment is a mode of emitting the light in synchronization with an instruction from the user (turning on a freeze switch), and leads to such a tendency that the light is emitted a smaller number of times per unit time compared to the "real-time mode" of consecutively carrying out the light irradiation. Therefore, the "freeze mode" leads to such a tendency that the heat generation amount per unit time does not largely increase even with an increase in the light quantity of one pulse compared to the "real-time mode". Therefore, in the present exemplary embodiment, the photoacoustic apparatus increases the irradiation light quantity in the case of the "freeze mode" compared to the irradiation light quantity in the "real-time mode".

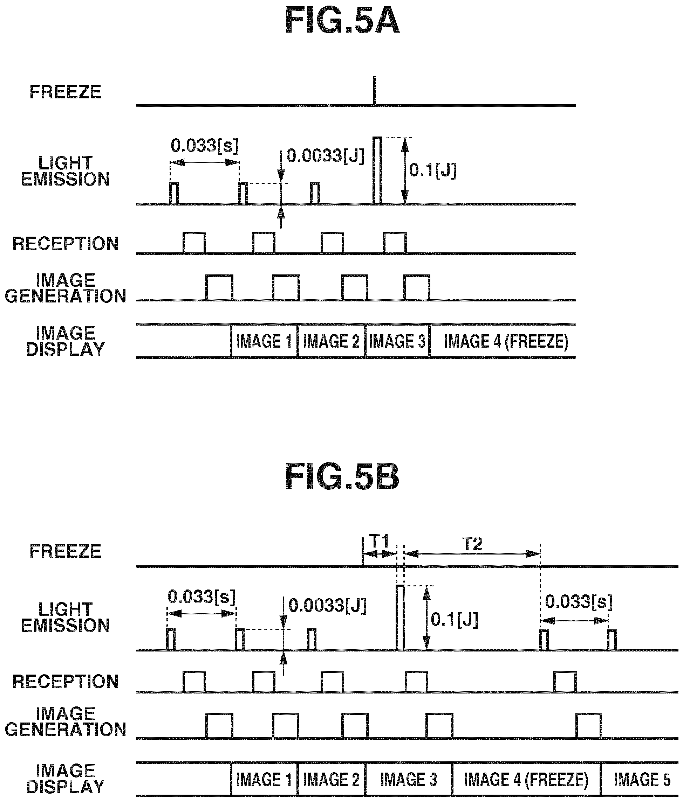

For example, the user can switch each of the modes to carry out the light irradiation as indicated in a timing chart illustrated in Fig. 5A. Fig. 5A illustrates an operation when the user issues an instruction for the freeze with use of the input unit 170 during the "high-speed real-time mode" described in the first exemplary embodiment. The control unit 153 receives information based on the user's instruction from the input unit 170 at a timing indicated by "freeze" (timing at which the user issues the instruction) illustrated in Fig. 5A. The control unit 153 transmits a signal for controlling the light irradiation corresponding to the "freeze mode" to the driver circuit 114 based on the instruction information. The driver circuit 114 supplies power corresponding to the "freeze mode" to the light source 111 based on the signal for controlling the light irradiation, and the light source 111 emits the light ("light emission" illustrated in Fig. 5A). In the "freeze mode" according to the present exemplary embodiment, the light source 111 emits the light by an irradiation light quantity of 0.1 [J]. For example, supposing that the freeze button is pressed once in 1 second, the average power is 0.1 [W] and the heat generation amount is 0.9 [W] in the "freeze mode". In this manner, in the "freeze mode", the photoacoustic apparatus can keep the heat generation amount per unit time under the predetermined value even when increasing the irradiation light quantity compared to the "high-speed real-time mode". Further, the photoacoustic apparatus improves the image quality of the displayed image compared to that in the "high-speed real-time mode" because increasing the irradiation light quantity in the "freeze mode".

The reception unit 120 receives the photoacoustic wave generated due to the light emitted from the light source 111 at a timing indicated by "reception" illustrated in Fig. 5A. The calculation unit 151 performs the reconstruction processing based on the signal output from the reception unit 120 to generate the image data at a timing indicated by "image generation" illustrated in Fig. 5A. Then, the control unit 153 transmits the image data to the display unit 160 to cause the display unit 160 to display thereon the image based on the image data. The display unit 160 displays the image based on the image data thereon for a time period indicated by "image display" illustrated in Fig. 5A.

More specifically, in the timing chart illustrated in Fig. 5A, first, the displayed image (images 1 to 3) is updated every 0.033 seconds, and the user issues the instruction for the freeze while the moving image is displayed. Then, a freeze image (image 4) acquired from the light irradiation based on the freeze instruction issued by the user is displayed after the instruction. More specifically, the freeze image (image 4) as a still image due to the light irradiation based on the user's instruction is displayed after the moving image (images 1 to 3) is displayed. The control unit 153 may transfer the image data acquired based on the freeze instruction and the displayed image based on this image data to the storage unit 152 or a picture archiving communication system (PACS) to store them.

However, the user issues the instruction for the freeze at an arbitrary timing, so that an instruction to release the freeze may be issued before a sufficient heat dissipation is completed. In such a case, the temperature inside the housing 181 may noticeably increase.

Therefore, the photoacoustic apparatus may carry out the light irradiation corresponding to the "freeze mode" after a time period T1 has elapsed since the user's issue of the instruction for the freeze as illustrated in Fig. 5B. In this case, the control unit 153 controls the driver circuit 114 so as to supply the power corresponding to the "freeze mode" to the light source 111 after the time period T1 has elapsed since the reception of the information based on the user's instruction. By performing such control, the photoacoustic apparatus can secure a time period required for the heat dissipation even when the freeze instruction is issued before the sufficient heat dissipation is completed. For example, the time period T1 may be set to a shorter time period than 0.1 seconds so that the user can feel as if the freeze image is displayed in real-time after issuing the freeze instruction. Alternatively, the time period T1 may be set to a shorter time period than 1 second to secure the sufficient time period for the heat dissipation.

Further, the photoacoustic apparatus may transition to the "real-time mode" again upon the completion of the sufficient heat dissipation after carrying out the light irradiation corresponding to the "freeze mode" as illustrated in Fig. 5B. More specifically, the control unit 153 may perform control so as to release the "freeze mode" and transition to the "real-time mode" after a time period indicated as a time period T2 has elapsed after the execution of the light irradiation corresponding to the "freeze mode". Due to this mode transition, the display on the display unit 160 is switched from the image 4, which is the still image, to the moving image starting from the image 5. For example, the user issues the freeze instruction when the user intends to confirm the freeze image in detail, so that the time period T2 may be set to a longer time period than 5 seconds. Alternatively, the time period T2 may be set to a shorter time period than 1 second in order to secure the sufficient time period for the heat dissipation. The control unit 153 may carry out the transition from the "freeze mode" to the "real-time mode" based on the user's instruction.

Then, if the user frequently issues the freeze instruction, the heat generation amount per unit time may undesirably increase. Therefore, the control unit 153 may limit the number of times that the light is emitted based on the freeze instruction per unit time. The time periods T1 and T2 in the "freeze mode" are set in such a manner that the irradiation light quantity determined in the "freeze mode" and the heat generation amount per unit time when the light is emitted according to a cycle determined by (T1 + T2) fall below the predetermined value (e.g., 1 [W]). Further, the control unit 153 may limit the number of times that the light is emitted based on the freeze instruction within the (T1 + T2) time period to once. This limitation may be realized, for example, with use of firmware configured to ignore the instruction from the user when the number of times that the freeze instruction is issued per unit time exceeds a predetermined value. Alternatively, the limitation on the light emission based on the freeze instruction may be realized by a method using hardware. For example, the light emission may be limited with use of a signal generated from logical AND of a clock signal of an upper limit value of a frequency at which the freeze instruction is repeated and a signal of the freeze switch by the user. Alternatively, a high-frequency component of the signal of the freeze switch may be removed with use of a low-pass filter constructed by an analog or digital circuit.

The control unit 153 may issue a notification prompting the limitation on the freeze instruction on the display unit 160 or the like when the number of times that the freeze instruction is issued per unit time reaches the predetermined value. For example, a unit that issues the notification with use of a display lamp, a sound/voice, and/or the like may be employed as a notification unit, besides a unit that presents a display prompting the limitation on the freeze instruction on the display unit 160.

In Figs. 5A and 5B, the photoacoustic apparatus has been described as the example in which the light irradiation using one pulse is carried out based on the freeze instruction issued by the user. However, in the present exemplary embodiment, the photoacoustic apparatus may carry out the light irradiation using a plurality of pulses based on the freeze instruction issued by the user and generate the freeze image with use of signal data acquired from the light irradiation using the plurality of pulses as the "freeze mode". In this case, the photoacoustic apparatus can also set the irradiation light quantity per pulse to a larger amount than that in the "real-time mode" of periodically carrying out the light irradiation.

The light irradiation unit 110 may include a plurality of light sources, and switch the light source 111 according to the mode. For example, the LD may be used as the light source 111 in the "freeze mode" in which the irradiation light quantity is large. On the other hand, the LED may be used in the "high-speed real-time mode" as the light source 111 in which the irradiation light quantity is small. By switching and using the light source capable of efficiently emitting the irradiation light quantity of each of the modes in this manner, the photoacoustic apparatus can efficiently use the supplied power, thereby leading to a reduction in the heat generation. Further, the photoacoustic apparatus can prevent the heat from being locally concentrated by switching the light source 111 according to the mode in this manner.

Further, the light irradiation unit 110 may expand the area to be irradiated with the light in the "high-speed real-time mode" while narrowing this area in the "freeze mode". In other words, the light irradiation unit 110 may increase the density of the light energy with which the subject 100 is irradiated by narrowing the irradiation area at the time of the mode requiring a large irradiation light quantity. As a result, the photoacoustic apparatus can acquire a high generated sound pressure of the photoacoustic wave in the "freeze mode", thereby further improving the image quality of the displayed image. Further, in the case where the image quality is supposed to be kept consistent between the modes, the photoacoustic apparatus may reduce the irradiation light quantity to reduce the heat generation in the "freeze mode".

Next, a fourth exemplary embodiment of the present invention will be described. The fourth exemplary embodiment is an exemplary embodiment using the LD, the LED, and/or the like as the light source 111, and especially useful in a case where the S/N of the photoacoustic signal due to the irradiation with one pulse is insufficient. One conceivable configuration in the case where the light emission of one pulse cannot satisfy a sufficient light quantity is to emit pulsed light a plurality of times, addition-average acquired individual photoacoustic signals to improve the S/N, and generate the photoacoustic image based on the addition-averaged photoacoustic signals. In this case, addition averaging processing such as simple averaging, moving averaging, and weighted averaging can be employed as the averaging.

The fourth exemplary embodiment is such an exemplary embodiment that the photoacoustic apparatus emits the pulsed light the plurality of times and addition-averages the acquired photoacoustic signals to acquire one reconstructed image. In the fourth exemplary embodiment, assume that a total light quantity of the pulsed light emission carried out the plurality of times to acquire one reconstructed image is handled in a similar manner to the above-described irradiation light quantity. The photoacoustic apparatus can employ the light irradiation condition according to the above-described first to third exemplary embodiments by handling the irradiation light in this manner. Further, in this case, the frequency at which the light irradiation is repeated does not correspond to a frequency defined from a time interval of emitting the pulsed light multiple times to addition-average the photoacoustic signals but corresponds to a frequency based on a cycle per which the reconstructed image is acquired (the refresh frequency).

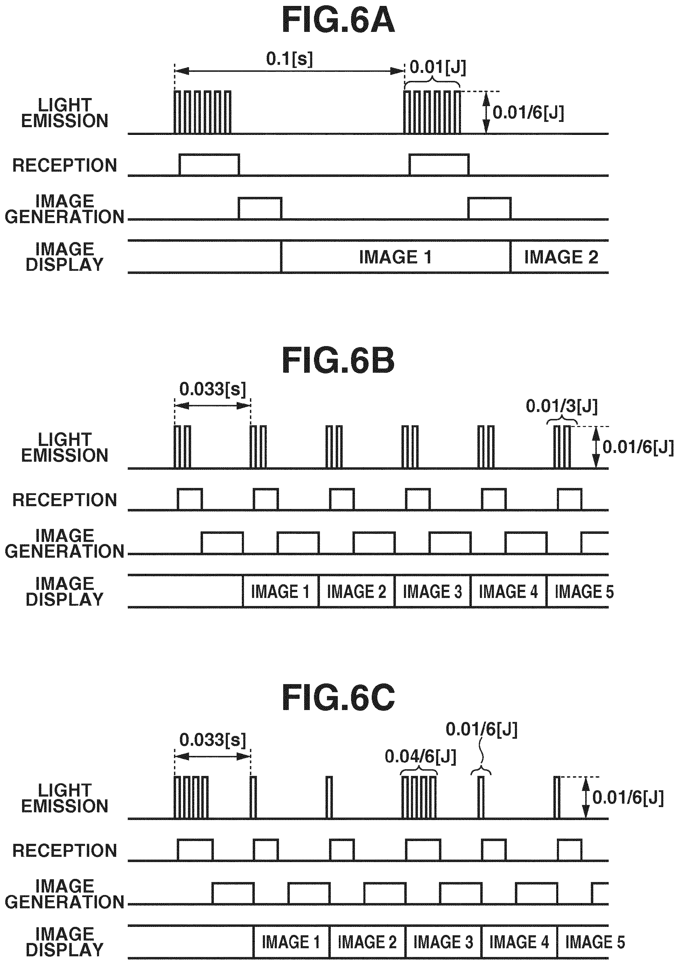

Figs. 6A, 6B, and 6C are timing charts illustrating a method for controlling the light source 111 and a heat generation amount per unit time according to the fourth exemplary embodiment. Descriptions of portions in Figs. 6A, 6B, and 6C that are similar to Figs. 4A and 4B will be omitted below.

Figs. 6A, 6B, and 6C illustrate each of the timings at which the irradiation light is emitted, the photoacoustic wave is received, the image data is generated, and the image data is displayed, similarly to Figs. 4A and 4B. Axes of ordinate in timing charts of "light emission" indicate a light quantity of each pulsed light in the pulsed light emission carried out the plurality of times. Further, the total light quantity (the irradiation light quantity) due to the pulsed light emission carried out the plurality of times is also indicated together therewith.

A difference from Figs. 4A and 4B is to emit the pulsed light the plurality of times, addition-average the acquired photoacoustic signals, and reconstruct the image based on the addition-averaged photoacoustic signals. In the case where the pulsed light is emitted the plurality of times in this manner, controlling the light quantity itself of each pulsed light emission operation in the pulsed light emission carried out the plurality of times leads to complication of the circuit. Therefore, in the fourth exemplary embodiment, the photoacoustic apparatus employs a method that fixes the light quantity of each pulsed light emission operation in the pulsed light emission carried out the plurality of times, and control the light quantity (irradiation light quantity) by controlling the number of times that the light is emitted in the pulsed light emission carried out the plurality of times.

Further, according to the above-described control of the light quantity (irradiation light quantity), the fixed light quantity is used as the light quantity itself of each pulsed light emission operation in the pulsed light emission carried out the plurality of times, so that a light quantity distribution (intensity of the light quantity) inside the subject 100 little changes according to each pulsed light emission operation in the pulsed light emission carried out the plurality of times. Therefore, the photoacoustic apparatus does not have to, for example, control a gain of the amplifier by the signal collection unit 140 for each pulsed light beam corresponding to the pulsed light emission carried out the plurality of times. In this manner, due to the above-described control of the irradiation light quantity, the photoacoustic apparatus can also save a trouble of performing cumbersome control such as the gain control.

Fig. 6A is a timing chart in the "real-time mode", which can present the display following the normal movement of the hand-held type probe 180, and it is assumed that the frequency at which the image display is refreshed is set to 10 [Hz].

The control unit 153 sets the light quantity setting value of 0.01 [J] to the driver circuit 114 at a timing indicated by "light emission" illustrated in Fig. 6A. The driver circuit 114 causes the light source 111 such as the LD and the LED to emit the light according to the number of times that the pulsed light is emitted corresponding to the setting value of the light quantity (e.g., pulsed light of 0.01/6 [J] is emitted 6 times at a time interval of 2 [msec]) based on a signal indicating a timing of emitting the light every 0.1 seconds from the control unit 153.

Then, the reception unit 120 receives each of the photoacoustic signals generated due to the pulsed light emission carried out the plurality of times from the light source 111 at a timing indicated by "reception" illustrated in Fig. 6A, and addition-averages them.

The calculation unit 151 performs the reconstruction processing based on the addition-averaged photoacoustic signals output from the reception unit 120 to generate the image data at a timing indicated by "image generation" illustrated in Fig. 6A. Then, the control unit 153 transmits the image data to the display unit 160 to cause the display unit 160 to display thereon the image based on the image data. The display unit 160 displays the image based on the image data thereon during a time period indicated by "image display" illustrated in Fig. 6A.

In the timing chart illustrated in Fig. 6A, first, the image 1 is displayed for 0.1 seconds, and, next, the image 2 is displayed for 0.1 seconds. This process is repeated, and the image display based on the new image data is updated every 0.1 seconds.

As described above, the heat generation amount of the hand-held type probe 180 per unit time is determined based on the irradiation light quantity (total light quantity due to the pulsed light emission carried out the plurality of times to acquire one reconstructed image) and the frequency at which the light irradiation is repeated (frequency based on the cycle per which the reconstructed image is acquired). In the case of Fig. 6A, it is assumed that the light conversion efficiency with respect to the supplied power is 10 [%]. In this case, the heat generation amount of the light source 111 per unit time is 0.09/6 [J] × 6 × (1/0.1) = 0.9 [W], assuming that the irradiation light quantity is 0.01 [J] (light quantity of the pulsed light emission carried out once 0.01/6 [J] × 6 times).

Fig. 6B is a timing chart in the "high-speed real-time mode". Compared to Fig. 6A, the light irradiation is repeated at a triple frequency, and the irradiation light quantity (number of times that the light is emitted) is different. The repetition frequency illustrated in Fig. 6B is 30 [Hz] that is three times as high as Fig. 6A, and the light source 111 emits the pulsed light twice at the time interval of 2 [msec] for each cycle of 0.033 seconds. The displayed image can be updated every 0.033 seconds, and therefore followability is also improved compared to Fig. 6A. On the other hand, the irradiation light quantity in Fig. 6B is 0.01/3 [J] (i.e., light quantity of the pulsed light emission per one time (0.01/6 [J]) × 2 times), i.e., is set to one-third of Fig. 6A. By such a setting, the heat generation amount of the light source 111 per unit time is 0.9 [W].

In the above-described manner, in the fourth exemplary embodiment, the user can select the "real-time mode" (first mode) in which the light irradiation is repeated at the frequency of 10 [Hz] (first repetition frequency). Further, the user can select the "high-speed real-time mode" (second mode) in which the light irradiation is repeated at the frequency of 30 [Hz] (second repetition frequency) higher than 10 Hz. Further, the user can switch the "real-time mode" and the "high-speed real-time mode". Further, when irradiating the subject 100 with the light at the first repetition frequency (10 [Hz]), the photoacoustic apparatus performs the control in such a manner that the light is emitted from the light source 111 by the first irradiation light quantity (light quantity of the pulsed light emission per one time (0.01/6 [J]) is emitted 6 times). Alternatively, when irradiating the subject 100 with the light at the second repetition frequency (30 [Hz]), the photoacoustic apparatus performs the control in such a manner that the light is emitted from the light source 111 by the second irradiation light quantity smaller than the first irradiation light quantity (light quantity of the pulsed light emission per one time (0.01/6 [J]) × 2 times).

As described above, it can be understood that the heat generation amount of the light source 111 per unit time can be controlled by the frequency at which the light irradiation is repeated and the irradiation light quantity. In this case, the frequency at which the light irradiation is repeated corresponds to the frequency based on the cycle per which the reconstructed image is acquired, and the irradiation light quantity corresponds to the value proportional to the number of times that the light is emitted (light quantity of the pulsed light emission per one time to acquire one reconstructed image × the number of times that the light is emitted).

In the above description, the photoacoustic apparatus has been described as being configured in such a manner that the same light quantity is set for each operation in the pulsed light emission carried out the plurality of times to acquire one reconstructed image (fixed value: 0.01/6 [J]). The present exemplary embodiment may be configured in such a manner that a different light quantity is set for each operation in the pulsed light emission carried out the plurality of times. In this case, the present exemplary embodiment can be realized by also handling the total light quantity due to the pulsed light emission carried out the plurality of times to acquire one reconstructed image in a similar manner to the above-described irradiation light quantity.

The frequency at which the light irradiation is repeated and the irradiation light quantity illustrated in Figs. 6A and 6B are one example, and may be other values optimized for the system.

Further, the frequency at which the light irradiation is repeated and the irradiation light quantity (number of times that the light is emitted) may be controlled as illustrated in Fig. 6C to realize the "high-speed real-time mode". In Fig. 6C, the frequency at which the light irradiation is repeated is three times as high as the "real-time mode" illustrated in Fig. 6A similarly to Fig. 6B, and the irradiation light quantity is changed for each cycle corresponding to the frequency at which the light irradiation is repeated to control the heat generation amount.

More specifically, as illustrated in Fig. 6C, the cycle per which the light irradiation is repeated is 0.033 [s], and the subject 100 is irradiated with an irradiation light quantity of 0.04/6 [J] (pulsed light of 0.01/6 [J] is emitted 4 times) and an irradiation light quantity of 0.01/6 [J] (pulsed light of 0.01/6 [J] is emitted once).

In this case, the reconstructed image of some frame has an improved S/N and the reconstructed image of another frame has a lower S/N according to the irradiation light quantity. By performing such control, the photoacoustic apparatus can also acquire the reconstructed image in which the S/N is less reduced while preventing or cutting down the reduction in the refresh frequency. Then, the photoacoustic apparatus can operate so as to select the image of the frame acquired from the large irradiation light quantity when acquiring the image as the still image. By performing such control, the photoacoustic apparatus can prevent or cut down the increase in the temperature of the probe 180 while restraining the reduction in the S/N of the reconstructed image even in the "high-speed real-time mode" in which the refresh frequency is set to a high frequency.

In the exemplary embodiment illustrated in Fig. 6C, the constant light quantity is set for each operation in the pulsed light emission carried out the plurality of times. As described above, the light quantity distribution (intensity of the light quantity) inside the subject 100 little changes according to each pulsed light emission operation in the pulsed light emission carried out the plurality of times. Therefore, a fixed value may be used as, for example, the gain of the amplifier of the signal collection unit 140 corresponding to each operation in the pulsed light emission carried out the plurality of times. Further, according to such light irradiation, there is brought about an advantage of being able to reconstruct the image under the same condition regardless of the number of times that the pulsed light is emitted, which is emitted the plurality of times, when addition-averaging the photoacoustic signals acquired from the individual operations in the pulsed light emission carried out the plurality of times.

The present exemplary embodiment may be applied to the configuration that emits the pulsed light once for each cycle corresponding to the frequency at which the light irradiation is repeated (cycle per which the reconstructed image is acquired), like the above-described first to third exemplary embodiments. In this case, the present exemplary embodiment may be realized by changing the light quantity of the pulsed light emission, making the gain of the amplifier of the signal collection unit 140 variable, and correcting a change in the photoacoustic signal due to the change in the light quantity of the pulsed light emission.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A photoacoustic apparatus comprising:
a hand-held type probe (180) including a light source (110), and a reception means (120) configured to receive a photoacoustic wave generated due to light irradiation of a subject with light emitted from the light source (110); and
a supply means (190) configured to supply power to the light source (110) in such a manner that a heat generation amount generated in the light source (110) per unit time does not exceed a predetermined value.

2. The photoacoustic apparatus according to claim 1, wherein the light source (110) includes at least one of a light-emitting diode (111) and a laser diode (111).

3. The photoacoustic apparatus according to claim 1,
wherein the supply means (190) supplies the power to the light source (110) in such a manner that the light is emitted from the light source (110) by a first irradiation light quantity when the subject is repeatedly irradiated with the light, and supplies the power to the light source (110) in such a manner that the light (110) is emitted from the light source by a second irradiation light quantity larger than the first irradiation light quantity when the subject is irradiated with the light at a timing determined based on an instruction for the light irradiation that is issued by a user.

4. The photoacoustic apparatus according to claim 3, further comprising an information processing means (150) configured to cause a display means (160) to display thereon an image with use of a reception signal that the reception means (120) outputs by receiving the photoacoustic wave,
wherein the information processing means (150) causes the display means (160) to display thereon a moving image with use of the reception signal of the photoacoustic wave generated due to the repetitive light irradiation of the subject by the first irradiation light quantity, and causes the display means (160) to display thereon a still image with use of the reception signal of the photoacoustic wave generated due to the light irradiation of the subject by the second irradiation light quantity at the timing determined based on the instruction for the light irradiation that is issued by the user.

5. The photoacoustic apparatus according to claim 3 or 4,
wherein the light source (110) includes a light-emitting diode (111) and a laser diode (111), and
wherein the supply means (190) supplies the power to the light-emitting diode (111) in such a manner that the light is emitted from the light-emitting diode (111) by the first irradiation light quantity when the subject is repeatedly irradiated with the light, and supplies the power to the laser diode (111) in such a manner that the light is emitted from the laser diode (111) by the second irradiation light quantity when the subject is irradiated with the light at the timing determined based on the instruction for the light irradiation that is issued by the user.

6. The photoacoustic apparatus according to claim 1,
wherein the supply means (190) supplies the power to the light source (110) in such a manner that the light is emitted from the light source (110) by a first irradiation light quantity when the subject is irradiated with the light at a first repetition frequency, and supplies the power to the light source in such a manner that the light is emitted from the light source (110) by a second irradiation light quantity larger than the first irradiation light quantity when the subject is irradiated with the light at a second repetition frequency lower than the first repetition frequency.

7. The photoacoustic apparatus according to claim 6, further comprising an information processing means (150) configured to cause a display means (160) to display thereon an image with use of a reception signal that the reception means (120) outputs by receiving the photoacoustic wave,
wherein the information processing means (150) causes the display means (160) to display thereon a moving image at a first refresh frequency with use of the reception signal of the photoacoustic wave generated due to the light irradiation of the subject at the first repetition frequency and by the first irradiation light quantity, and causes the display means (160) to display thereon a moving image at a second refresh frequency lower than the first refresh frequency with use of the reception signal of the photoacoustic wave generated due to the light irradiation of the subject at the second repetition frequency and by the second irradiation light quantity.

8. The photoacoustic apparatus according to claim 6 or 7,
wherein the light source (110) includes a light-emitting diode (111) and a laser diode (111), and
wherein the supply means (190) supplies the power to the light-emitting diode (111) in such a manner that the light is emitted from the light-emitting diode (111) by the first irradiation light quantity when the subject is irradiated with the light at the first repetition frequency, and supplies the power to the laser diode (111) in such a manner that the light is emitted from the laser diode (111) by the second irradiation light quantity when the subject is irradiated with the light at the second repetition frequency.

9. A method for controlling power to be supplied to a light source applied to a hand-held type probe (180) including the light source (110) and a reception means (120) configured to receive a photoacoustic wave generated due to light irradiation of a subject with light emitted from the light source, the method comprising:
controlling the power to be supplied to the light source (110) in such a manner that a heat generation amount generated in the light source per unit time does not exceed a predetermined value.

10. The method according to claim 9, further comprising:
causing the light source (110) to emit the light by a first irradiation light quantity when repeatedly irradiating the subject with the light; and
causing the light source (110) to emit the light by a second irradiation light quantity larger than the first irradiation light quantity when irradiating the subject with the light at a timing determined based on an instruction for the light irradiation that is issued by a user.

11. The method according to claim 10, further comprising:
displaying a moving image based on the photoacoustic wave generated due to the repetitive light irradiation of the subject by the first irradiation light quantity; and
displaying a still image with use of a reception signal of the photoacoustic wave generated due to the light irradiation of the subject by the second irradiation light quantity at the timing determined based on the instruction for the light irradiation that is issued by the user.

12. The method according to claim 9, further comprising:
causing the light source (110) to emit the light by a first irradiation light quantity when irradiating the subject with the light at a first repetition frequency; and
causing the light source (110) to emit the light by a second irradiation light quantity larger than the first irradiation light quantity when irradiating the subject with the light at a second repetition frequency lower than the first repetition frequency.

13. The method according to claim 12, further comprising:
displaying a moving image at a first refresh frequency with use of a reception signal of the photoacoustic wave generated due to the light irradiation of the subject at the first repetition frequency and by the first irradiation light quantity; and
displaying a moving image at a second refresh frequency lower than the first refresh frequency with use of a reception signal of the photoacoustic wave generated due to the light irradiation of the subject at the second repetition frequency and by the second irradiation light quantity.

14. A program that when executed on a computer causes the computer to perform the control method according to any one of claims 9 to 13.
